# EUROPEAN PATENT APPLICATION

(11) **EP 1 469 290 A1**
(43) Date of publication of application: **20.10.2004**
(21) Application number: 04252130.2
(22) Date of filing: 08.04.2004
(51) Int. Cl.: G01J 1/42, G02B 5/28, C02F 1/32

(54) **Ultraviolet sensors for monitoring an ultraviolet lamp in the germicidal wavelengths range**

(30) Priority: 14.04.2003 US 412215
(71) Applicant: GENERAL ELECTRIC COMPANY, Schenectady, NY 12345 (US)
(72) Inventor: Brown, Dale Marius, Schenectady, New York 12309 (US); Sandvik, Peter Micah, Guilderland, New York 12084 (US); Matocha, Kevin, Troy, New York 12180 (US); Lombardo, Leo, Lyndhurst, Ohio 44124 (US)
(74) Representative: Goode, Ian Roy

(57) **Abstract**

An ultraviolet sensor (10) monitors an effectiveness of ultraviolet lamps used in sterilization systems. the sensor includes an ultraviolet photodetector (12) and a filter (14) cooperating therewith configured for detecting light at wavelengths between 200-300 nm. a purification system for air or water utilizes the sensor in conjunction with an ultraviolet lamp directing ultraviolet light toward the air or water.

## Description

The invention relates to ultraviolet detectors with tailored response to sense effective wavelengths for germicidal applications.

Drinking water, industrial water, pure-water and wastewater treatment facilities along with air purifiers use high-power lamps which emit in the ultraviolet wavelengths to sterilize potential harmful micro-organisms in the water or air flowing past the lamps. In order to ensure the effectiveness of the ultraviolet light in sterilizing micro-organisms in the water or air, the output power of the lamps must be sensed. These sensors allow for control of the lamp output power to reduce energy consumption, to micro-organism sterilization, and to determine lamp replacement requirements.

The effective germicidal wavelengths for ultraviolet light generally range from 200 to 300 nanometers (nm) with a maximum effectiveness at 265 nm. The lamps used to provide this energy typically produce energy over a much broader spectrum exceeding 600 nm. In order to insure adequate energy for germicidal efficacy, sensor measurements should be limited to the energy in the effective wavelengths. The problem was to develop a sensing element that would respond only to the required germicidal wavelengths and that would exhibit long life under the intense ultraviolet energy required.

A significant amount of the lamp's spectral intensity is above 300 nm. Therefore, it is desirable to limit the responsivity of the detector to wavelengths less than 300 nm. Because the lamps can degrade over time or become fouled and the total output can decrease in the short wavelength region, it is important to monitor the light intensity below 300 nm. Otherwise, sterilization could become incomplete.

Present wastewater treatment systems use silicon or silicon carbide (SiC) photodetectors which are sensitive to a broad range of ultraviolet light to sense the amount of optical power that penetrates the treated material (water or air) in order to control the output power of the lamps. This control of the lamps is used to minimize energy costs, to ensure sterilization of bacteria, and to determine when lamp replacement is required.

Present silicon sensors detect energy over a broad range of wavelengths broader than the effective sterilization wavelengths, peaking in the infrared at about 1000 nm. This broad response places a challenging attenuation requirement on any attached filter since the sensitivity in the infrared is much higher than in the ultraviolet range. Additionally, these devices exhibit very short life expectancy due to rapid deterioration under intense ultraviolet light. This is most likely due to the fact that in order to detect ultraviolet (UV) light, Si detectors require the addition of a phosphor. These phosphors degrade under the high intensities generated by the UV lamps as required for effective sterilization of bacteria.

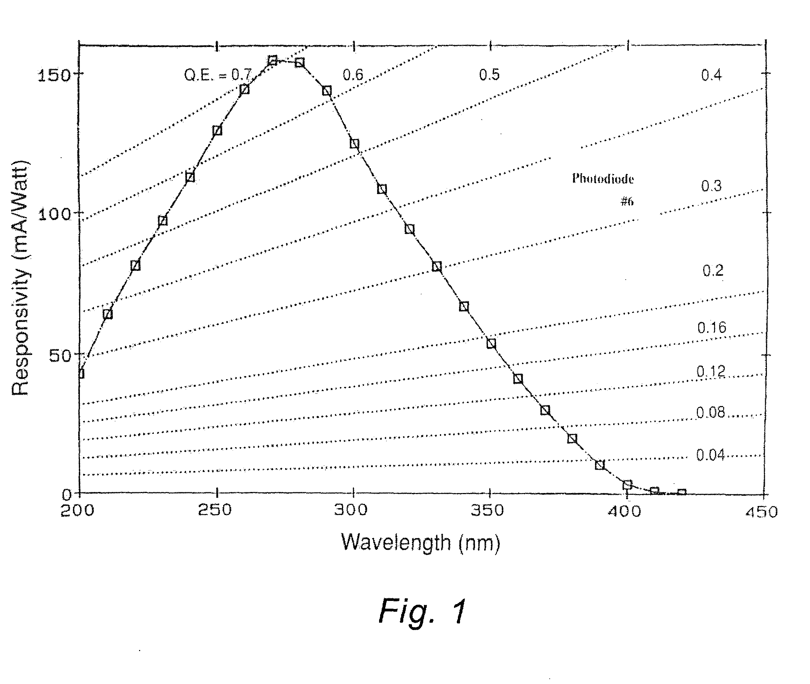

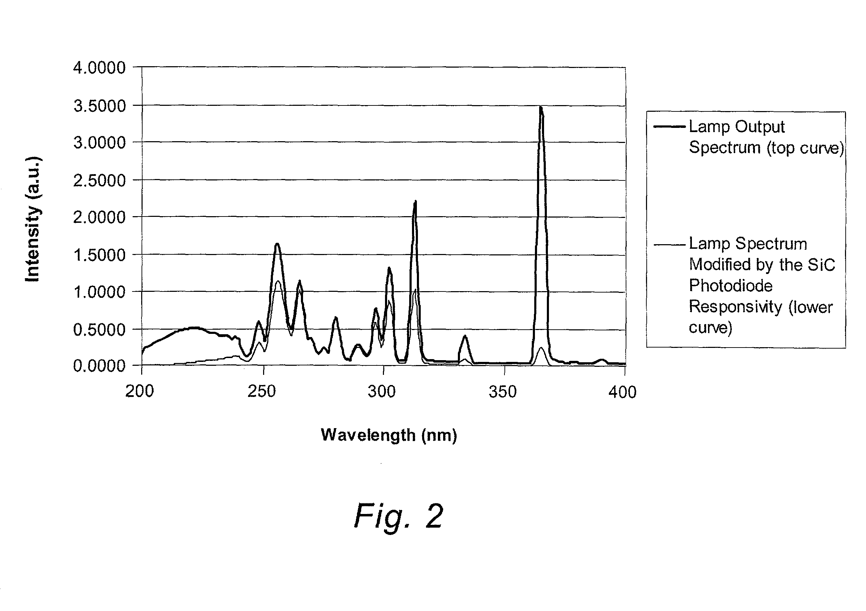

The advent of SiC photodiodes provided a more effective method of monitoring this ultraviolet energy without a filter. The spectral response of SiC photodiodes is largely confined to 200 to 400 nm with a peak around 270 nm (Fig. 1). SiC photodiodes have been demonstrated to have a very long life under intense ultraviolet light. However, the UV light spectrum outside the germicidal wavelengths (from 300 to 400 nm) may contain strong energy peaks from the lamps that degrade the monitoring accuracy of the intensity of the germicidally-effective wavelengths. The SiC photodiode's responsivity shown in Fig. 1 does already reduce the SiC photodiodes sensitivity to the lamp's output above 300 nm as shown in Fig. 2. However, the SiC photodiode will still produce a significant output photocurrent for those wavelengths between 300 and 400 nm. It would be desirable, therefore, to eliminate responsivity to wavelengths above 300 nm.

In an exemplary embodiment of the invention, an ultraviolet sensor is provided for monitoring energy in predetermined wavelengths for sterilizing microorganisms. The sensor includes an ultraviolet photodetector sensitive to a broad range of ultraviolet light, and a filter disposed in a position to intercept light directed toward the ultraviolet photodetector. The filter is configured to block light at wavelengths outside of the predetermined wavelengths.

In another exemplary embodiment of the invention, an ultraviolet sensor for monitoring an effectiveness of ultraviolet lamps used in sterilization systems includes an ultraviolet photodetector and a filter cooperating therewith configured for detecting light at wavelengths between 200-300 nm.

In still another exemplary embodiment of the invention, a purification system for air or water includes an ultraviolet lamp directing ultraviolet light toward the air or water, and the ultraviolet sensor of the invention.

In yet another exemplary embodiment of the invention, a method of purifying air or water includes the steps of directing ultraviolet light toward the air or water with an ultraviolet lamp; providing an ultraviolet sensor comprising an ultraviolet photodetector and a filter cooperating therewith configured for detecting light at wavelengths between 200-300 nm; and monitoring an effectiveness the ultraviolet lamp according to signals from the ultraviolet sensor.

Embodiments of the invention will now be described, by way of example, with reference to the accompanying drawings, in which:
FIGURE 1 is a graph showing conventional silicon carbide photodiode responsivity versus wavelength;
FIGURE 2 is a graph showing the lamp spectrum modified by the SiC photodiode responsivity;
FIGURE 3 shows a schematic cross section of a silicon carbide photodiode with a multiple layered dielectric filter applied to the top surface;
FIGURE 4 illustrates an alternative construction of the detector with the filter separated from the photodiode;
FIGURE 5 is a graph showing a prediction for optimized filter transmission characteristics;
FIGURE 6 is a graph showing the effect of the filter on the SiC photodiode's responsivity to eliminate the response to lamp emissions above 300 nm;
FIGURE 7 is a control loop schematic showing an exemplary application of the detector;
FIGURE 8 is a schematic of the photodiode housing showing the addition of an ultraviolet light source such as a UV LED used to periodically test the photodiode for functionality; and
FIGURE 9 shows the use of a movable reflective shutter to test the transparency of the sensor window.

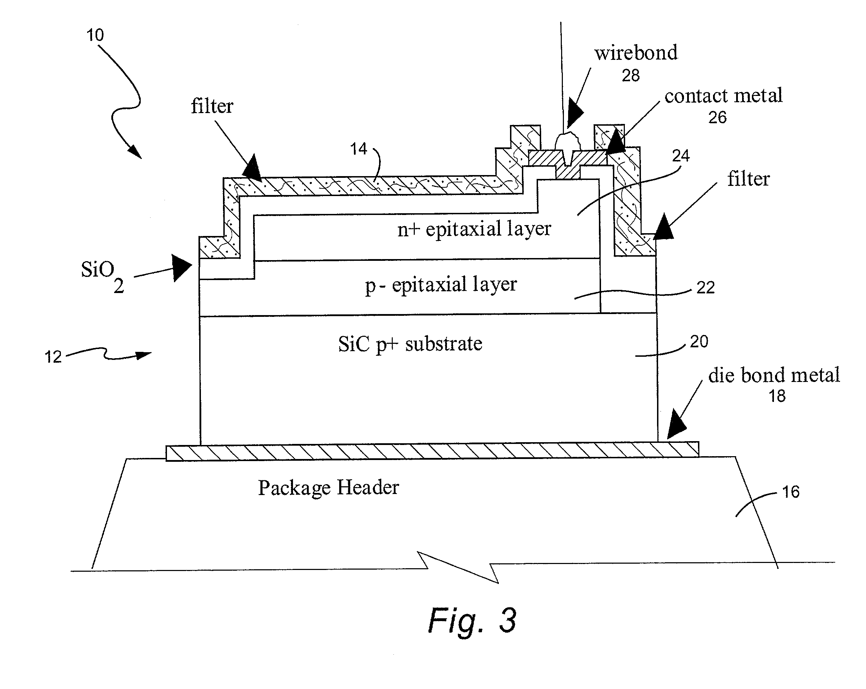

FIG. 3 shows a schematic cross section of the detector 10 of the invention including a SiC photodiode 12 with a multiple dielectric filter 14 applied to the top surface. The dielectric filter 14 is preferably deposited on the surface of the SiC photodiode 12 to tailor its response for efficient monitoring of specific spectral bands in the ultraviolet range. The use of silicon carbide. and deposited filter(s) will provide a robust detector 10 capable of long life under intense ultraviolet radiation. In FIG. 3, a cross-section of an example SiC photodiode is presented. Here, a package header 16 serves as a mount for the photodetector chip. A die bond metal 18 is used to keep the photodetector in place. A SiC substrate 20 and epitaxial layers 22, 24 comprise the semiconductor portion of this photodetector example. Here, n⁺ (negative) 24 and p⁻ (positive) 22 epitaxial layers serve to efficiently collect photogenerated carriers resulting from ultraviolet light of the pre-specified wavelengths. The optical filter 14 may consist of the prementioned materials, and in this embodiment, is an integral part of the chip. Contact metals 26 consisting at least partly of Au serve to provide low resistance contacts to the device, and are contacted to the photodiode package leads through using wirebond 28 to these contacts, also typically Au.

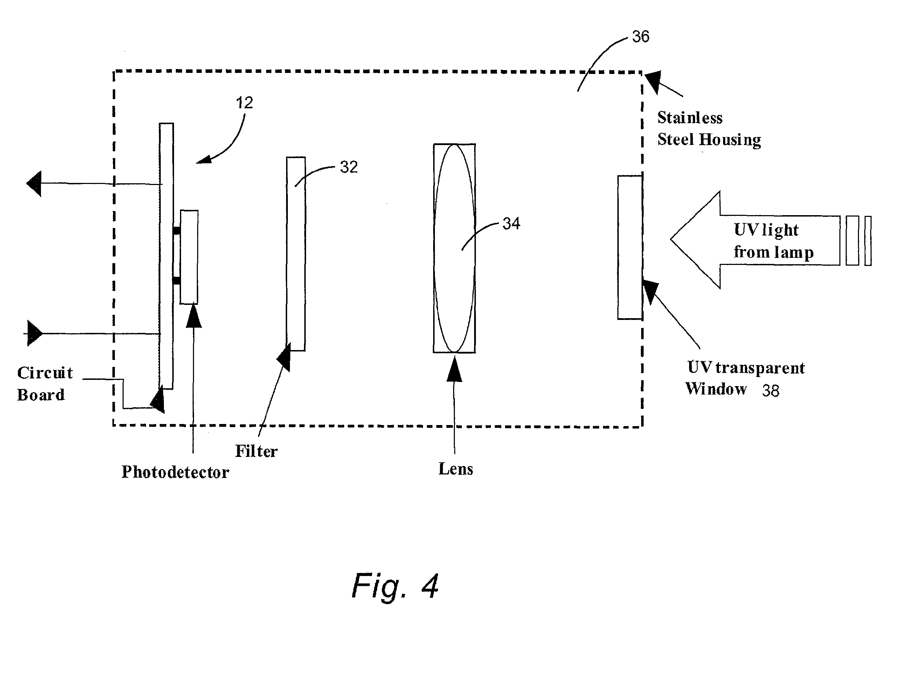

Preferably, any known sputtering technique may be used for depositing the dielectric filter, and thus the details of the deposition process will not be further described. Other suitable deposition methods may also be apparent to those of ordinary skill in the art, and the invention is not meant to be limited to the described exemplary application. Alternatively, with reference to FIG. 4, a separate filter 32 not attached to the photodiode could be placed in front of the SiC photodiode 12 receiving light input via a lens 34, with the components contained within a housing 36 having a UV transparent window 38. This arrangement, however, although viable, does not take advantage of integration, which allows for the simultaneous production of a plurality photodiodes with the integral filter in place. The production of photodiodes with integral filters can be easily accomplished by including the filter in the processing sequence. For example, ~1000 devices or more may be coated in one deposition in the case of dielectric materials, thereby greatly reducing the cost of the final device.

In a separated construction, the filter material is preferably deposited on a UV transparent substrate such as quartz or sapphire and then either cut to size or used in its entirety and inserted in the optical path.

Yet another embodiment may utilize a much more expensive, complex and much less practical optical spectrometer or photospectrometer, with or without an optical fiber input, and/or a photomultiplier tube. These options would require either a filter or software to determine the lamp's power in the range of interest between 200 and 300 nm.

Silicon carbide is particularly suited for the photodiode 12 since its response curve covers the spectrum of interest. An alternate photodiode material could be that of AlGaN, which can be made to have a shorter wavelength cutoff. For instance, GaN photodiodes have a cutoff at 365 nm. The addition of about 26% of Al to make an AlGaN photodiode could produce a cutoff of 300 nm. Quality AlGaN photodiodes, however, are not currently commercially available. SiC photodiodes, in contrast, are well established and readily available. The SiC photodiode is preferred at this time, although in the future, AlGaN photodiodes could perform the same function with or possibly without a filter. Still other materials may be suitable for the photodiode, such as silicon, gallium arsenide phosphode (GaAsP), zinc oxide (ZnO₂), aluminum nitride (AIN), gallium nitride (GaN), aluminum indium gallium nitride (AllnGaN), and indium gallium nitride (lnGaN). Alternatively, the ultraviolet photodetector may be a photomultiplier tube. Preferably, a responsivity of the combined ultraviolet photodetector and filter corresponds to an effectiveness of ultraviolet sterilization of microorganisms specific to a particular medium, such as water or air. Alternatively still, a photospectrometer may be used, which would not require such an optical filter as previously described. In this case, the spectrometer provides an array of photodiodes with each photodiode sensing specific wavelengths. A photospectrometer, however, is considerably more expensive than a semiconductor-based photodetector, thus semiconductor photodetectors are preferred.

The filter 14 is preferably a short wavelength pass filter that would cutoff at 300 nm. In one embodiment, the filter comprises a multiple-layer, dielectric filter composed of thin alternating layers of SiO₂, HfO2, SiO₂ and/or Si₃N₄. Other combinations of materials may also be suitable. The filter 14 can also be fabricated with narrow bandwidth characteristics to monitor groups of spectral lines of ultraviolet lamps. Such a selective band-pass filter would preferably be centered at 254 nm for instance. The 254 nm line is an intense line from a Hg arc lamp. Filters using rare earth doped glass (Shott filters) or semiconductor materials such as GaAsP, ZnO₂, AllnGaN, GaN, AlGaN, InGaN, AIN or combinations thereof might also be utilized.

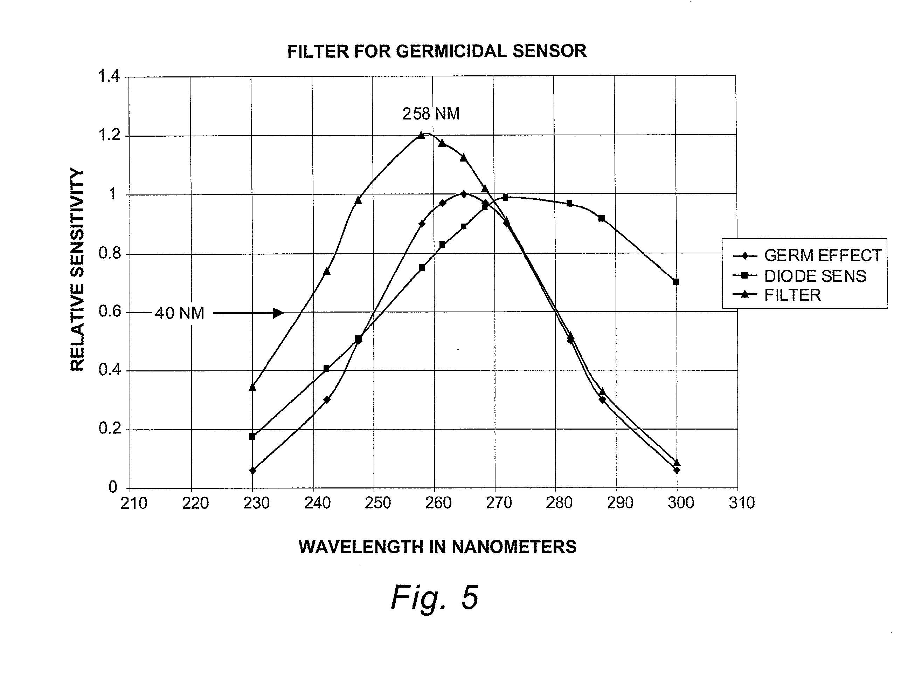

FIG. 5 shows optimized filter transmission characteristics based on the sensitivity (responsivity) of a typical SiC photodiode (square dot curve) and the effective wavelength band for light (radiation) suitable for sterilization of bacteria typical to these systems (diamond dot curve). A prediction for the optimized filter characteristics (triangle dot curve) takes the typical light output from a high intensity mercury lamp (center radiation at 254 nm) and allows the photodiode to respond only to the most effective kill band (centered at 265 nm).

This is one embodiment of an "optimized" design, which suggests that the best responsivity curve has a center response wavelength near 258 nm. Of course, any filter which blocks radiation above 300 nm may be suitable when placed in the optical path in front of the SiC photodiode whose responsivity falls rapidly below 270 nm.

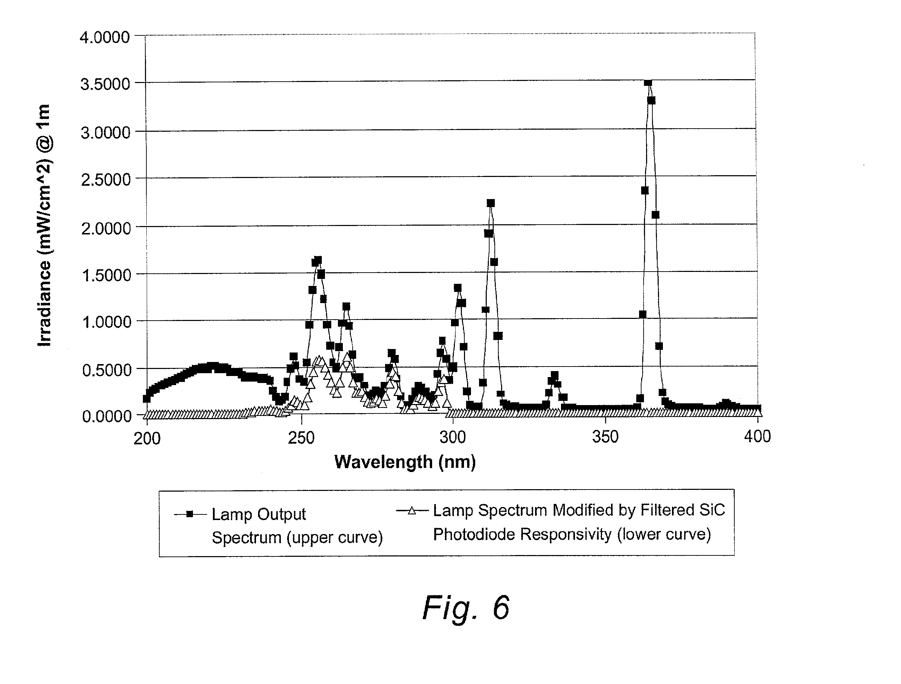

The square dot curve in FIG. 6 shows the output of a typical mercury lamp spectra. Note the emission peak at 254 nm. One concept of a filter which blocks radiation above 300 nm has been simulated (triangle dot curve), which eliminates the sensitivity (as sensed by the photodiode) for light above 300 nm. This light (above 300 nm) is essentially useless and would not be beneficial for assessing the condition of the lamp, i.e., its effectiveness in killing/sterilizing bacteria.

The filtered SiC photodiode is connected to signal conditioning circuitry to provide current, voltage frequency or digital output as required by the specific application.

Light from the sterilization lamps passes through the medium to be sterilized (water or air) and impinges upon the filter and is then measured by the detector 10. The combination of the filter and sensor measures only the wavelengths of light which are effective in sterilizing micro-organisms. A current to voltage amplifier, whose gain is determined by a feedback network amplifies the photodiode signal. This network can provide adjustable gain for calibration. Output from the amplifier can be converted to an industry standard current output or to a voltage, frequency or digital output as required.

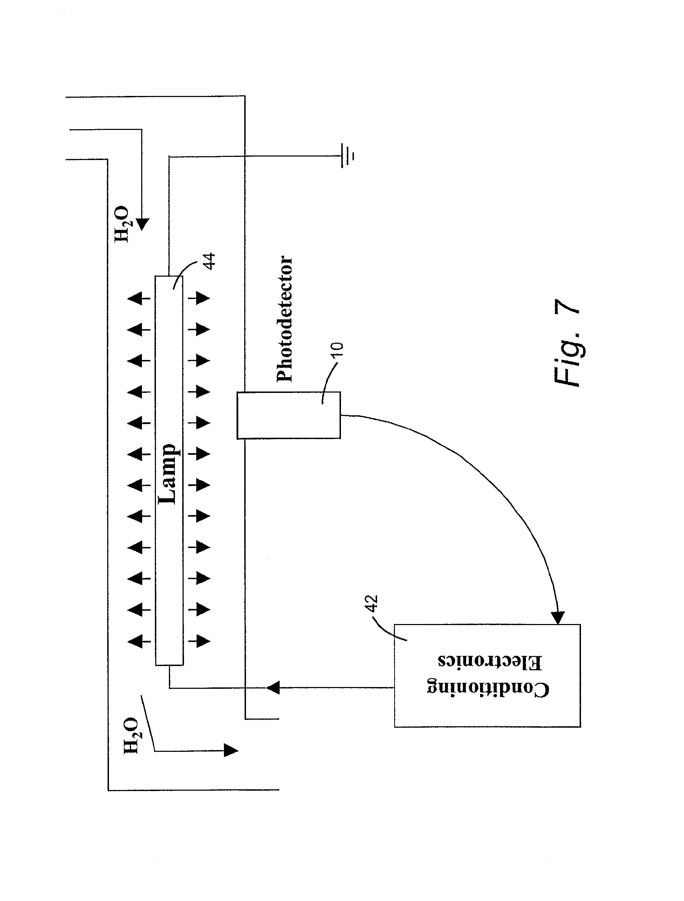

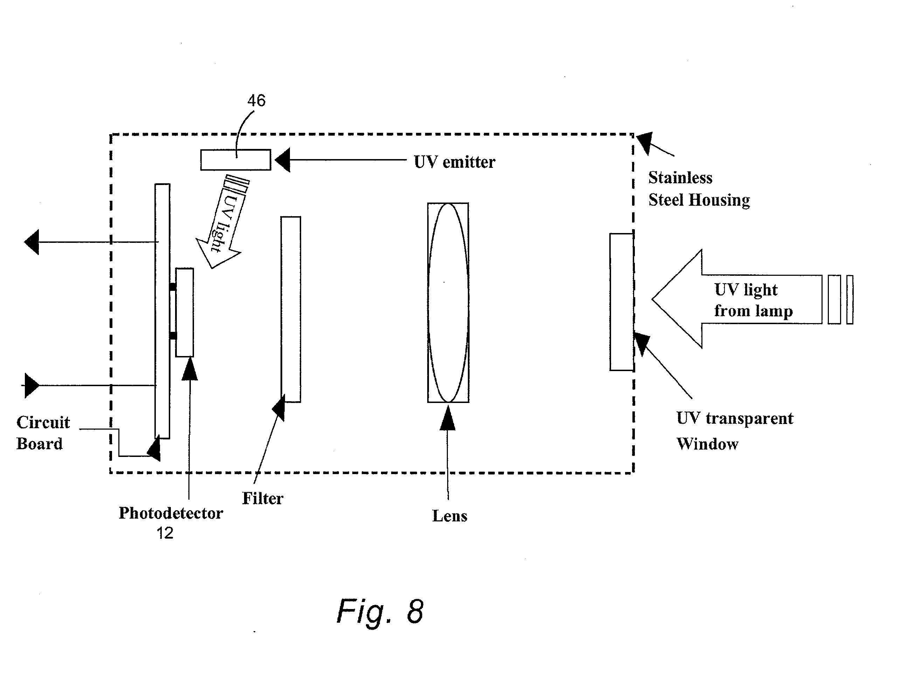

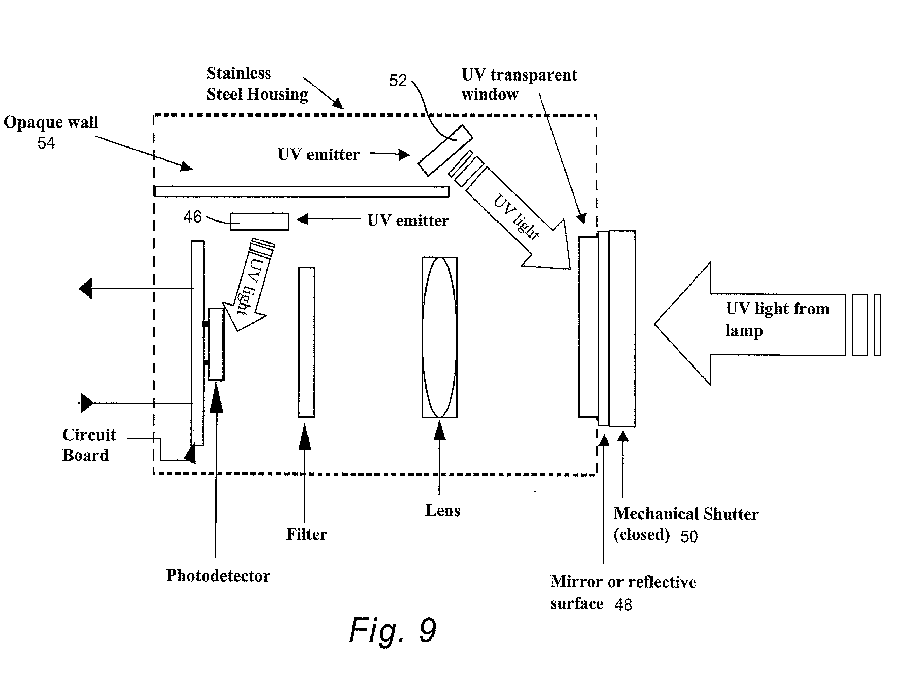

In one embodiment, with reference to FIG. 7, the detector 10 is utilized as part of a control loop including a processor 42, such as a CPU or the like, and the ultraviolet lamp 44. The processor 42 receives signals from the ultraviolet sensor 10 and controls an output of the ultraviolet lamp 44 based on the ultraviolet sensor signals. In this manner, the effectiveness of the ultraviolet lamp 44 can be monitored and lamp output can be controlled in real time. Moreover, with reference to FIG. 8, an additional ultraviolet light source 46 emitting light between 200 to 400 nm may be employed such that its emission would be sensed by the photodiode 12. This UV light source 46 would be used to occasionally test the photodetector, and determine its functionality over the course of time. The additional UV light source 46 could be for example a UV LED device, which could test the photodiode with non-integral or integral filter. In addition, as shown in FIG. 9, a reflective movable plate 48 and shutter 50 could be mounted just outside the window in combination with still another UV emitter 52 and opaque wall 54 in order to test for window coatings.

The sensor of the invention is suitable in the ultraviolet sterilization industry to monitor the amount of energy provided in the germicidal spectrum. The sensor ensures that enough energy, at the appropriate wavelength, is always available for efficient sterilization. The signal can be used both to control the lamp output and to alarm of inadequate ultraviolet levels.

For completeness, various aspects of the invention are set out in the following numbered clauses:
1. An ultraviolet sensor for monitoring energy in predetermined wavelengths for sterilizing microorganisms, the sensor comprising:
   an ultraviolet photodetector (12) sensitive to a broad range of ultraviolet light; and
   a filter (14, 32) disposed in a position to intercept light directed toward the ultraviolet photodetector, the filter being configured to block light at wavelengths outside of said predetermined wavelengths.
2. A sensor according to clause 1, wherein the filter (14, 32) is configured to permit passage of light at wavelengths between 200 and 300 nm.
3. A sensor according to clause 2, wherein the ultraviolet photodetector (12) is a photodiode.
4. A sensor according to clause 3, wherein the ultraviolet photodetector (12) is a silicon carbide photodiode.
5. A sensor according to clause 3, wherein the ultraviolet photodetector (12) is a photodiode of a material selected from the group consisting of silicon, gallium arsenide phosphode (GaAsP), zinc oxide (ZnO₂), aluminum nitride (AIN), aluminum gallium nitride (AlGaN), gallium nitride (GaN), aluminum indium gallium nitride (AllnGaN), and indium gallium nitride (lnGaN).
6. A sensor according to clause 2, wherein the ultraviolet photodetector (12) is a photomultiplier tube.
7. A sensor according to clause 1, wherein the filter (14, 32) is a bandpass filter with a bandpass region from about 220 nm to 300 nm.
8. A sensor according to clause 1, where a responsivity of the combined ultraviolet photodetector (12) and filter (14, 32) corresponds to an effectiveness of ultraviolet sterilization of microorganisms specific to a particular medium.
9. A sensor according to clause 8, wherein the medium for sterilization is water.
10. A sensor according to clause 8, wherein the medium for sterilization is air.
11. A sensor according to clause 1, wherein the filter (14) is formed as an integral component of the ultraviolet photodetector (12) by being deposited on the ultraviolet photodetector.
12. A sensor according to clause 1, wherein the filter (32) is external to the ultraviolet photodetector (12).
13. A sensor according to clause 1, wherein the filter (14, 32) is formed of dielectric materials.
14. A sensor according to clause 13, wherein the dielectric materials are selected from the group comprising SiO₂, HfO₂, Si₃N₄ or combinations thereof.
15. A sensor according to clause 1, wherein the filter (14, 32) is formed of rare earth doped glass.
16. A sensor according to clause 1, wherein the filter (14, 32) is formed of semiconductor materials.
17. A sensor according to clause 16, wherein the semiconductor materials are selected from the group comprising GaAsP, ZnO₂, SiC, AllnGaN, AlGaN, GaN, InGaN, AIN or combinations thereof.
18. An ultraviolet sensor for monitoring an effectiveness of ultraviolet lamps used in sterilization systems, the ultraviolet sensor comprising an ultraviolet photodetector (12) and a filter (14, 32) cooperating therewith configured for detecting light at wavelengths between 200-300 nm.
19. A purification system for air or water comprising:
   an ultraviolet lamp (44) directing ultraviolet light toward the air or water;
      an ultraviolet sensor (10) for monitoring an effectiveness the ultraviolet lamp in predetermined wavelengths, the ultraviolet sensor comprising an ultraviolet photodetector (12) sensitive to a broad range of ultraviolet light, and
   a filter (14, 32) disposed in a position to intercept light directed toward the ultraviolet photodetector, the filter being configured to block light at wavelengths outside of said predetermined wavelengths.
20. A purification system according to clause 19, further comprising a processor (42) receiving signals from the ultraviolet sensor (10) and controlling an output of the ultraviolet lamp (44) based on the ultraviolet sensor signals.
21. A purification system according to clause 19, further comprising an additional ultraviolet light source (46) emitting light between 200 to 400 nm for testing the ultraviolet sensor (10).
22. A method of purifying air or water comprising:
   directing ultraviolet light toward the air or water with an ultraviolet lamp (44);
   providing an ultraviolet sensor (10) comprising an ultraviolet photodetector (12) and a filter (14, 32) cooperating therewith configured for detecting light at wavelengths between 200-300 nm; and
   monitoring an effectiveness the ultraviolet lamp according to signals from the ultraviolet sensor.
23. A method according to clause 22, further comprising controlling an output of the ultraviolet lamp (44) based on the ultraviolet sensor signals.
24. A sensor for monitoring energy in predetermined wavelengths for sterilizing microorganisms, the sensor comprising:
   a photospectrometer with photodetectors collecting light specifically in the predetermined wavelengths; and
   a software program capable of distinguishing light outside the predetermined wavelengths.

## Claims

1. An ultraviolet sensor for monitoring energy in predetermined wavelengths for sterilizing microorganisms, the sensor comprising:
an ultraviolet photodetector (12) sensitive to a broad range of ultraviolet light; and
a filter (14, 32) disposed in a position to intercept light directed toward the ultraviolet photodetector, the filter being configured to block light at wavelengths outside of said predetermined wavelengths.

2. A sensor according to claim 1, wherein the filter (14, 32) is configured to permit passage of light at wavelengths between 200 and 300 nm.

3. A sensor according to claim 1, where a responsivity of the combined ultraviolet photodetector (12) and filter (14, 32) corresponds to an effectiveness of ultraviolet sterilization of microorganisms specific to a particular medium.

4. A sensor according to claim 1, wherein the filter (14) is formed as an integral component of the ultraviolet photodetector (12) by being deposited on the ultraviolet photodetector.

5. A sensor according to claim 1, wherein the filter (32) is external to the ultraviolet photodetector (12).

6. A sensor according to claim 1, wherein the filter (14, 32) is formed of dielectric materials.

7. A sensor according to claim 1, wherein the filter (14, 32) is formed of semiconductor materials.

8. A purification system for air or water comprising:
an ultraviolet lamp (44) directing ultraviolet light toward the air or water;
an ultraviolet sensor (10) according to any one of claims 1 to 8.

9. A purification system according to claim 8, further comprising a processor (42) receiving signals from the ultraviolet sensor (10) and controlling an output of the ultraviolet lamp (44) based on the ultraviolet sensor signals.

10. A method of purifying air or water comprising:
directing ultraviolet light toward the air or water with an ultraviolet lamp (44);
providing an ultraviolet sensor (10) comprising an ultraviolet photodetector (12) and a filter (14, 32) cooperating therewith configured for detecting light at wavelengths between 200-300 nm; and
monitoring an effectiveness the ultraviolet lamp according to signals from the ultraviolet sensor.
